# FASCICULE DE BREVET EUROPEEN

(11) **EP 3 042 692 B1**
(45) Date de publication et mention de la délivrance du brevet: **22.11.2017**
(21) Numéro de dépôt: 15191385.2
(22) Date de dépôt: 26.10.2015
(51) Int. Cl.: A61N 1/05, A61N 1/08, A61N 1/36, A61N 1/362, A61N 1/368, A61N 1/37, A61N 1/39

(54) **DISPOSITIF MÉDICAL IMPLANTABLE POUR DES THÉRAPIES CARDIAQUE ET PÉRIPHÉRIQUE AVEC DES MOYENS POUR PERMETTRE OU POUR INHIBER LES THÉRAPIES SUR LA BASE DE SIGNAUX CAPTÉS**
IMPLANTIERBARE MEDIZINISCHE VORRICHTUNG ZUR BEHANDLUNG DES HERZENS UND DER PERIPHERIE MIT MITTELN ZUR ZULASSUNG ODER UNTERDRÜCKUNG DER BEHANDLUNG AUF BASIS VON ERFASSTEN SIGNALEN
IMPLANTABLE MEDICAL DEVICE FOR CARDIAC AND PERIPHERAL THERAPY WITH MEANS FOR ALLOWING OR INHIBITING THERAPY BASED ON SENSED SIGNALS

(30) Priorité: 19.11.2014 FR 1461187
(43) Date de publication de la demande: 13.07.2016
(73) Titulaire: Sorin CRM SAS, 92140 Clamart Cedex (FR)
(72) Inventeur: BLUMSTEIN, Hervé, 28130 Mévoisins (FR); MEVEL, Hervé, 1450 Chastre (St-Géry) (BE)
(74) Mandataire: Grünecker Patent- und Rechtsanwälte PartG mbB

(56) Documents cités:
- US-A1- 2011 093 026
- US-A1- 2011 224 988
- US-A1- 2012 123 496
- US-A1- 2013 116 743
- US-A1- 2014 330 328

## Description

L'invention concerne les dispositifs médicaux implantables actifs, tels que définis par la directive 90/385/CEE du 20 juin 1990 du Conseil des communautés européennes.

Ces dispositifs comprennent un générateur implantable contenant dans un même boitier les divers circuits électroniques, leur pile d'alimentation et une tête de connecteur pour le couplage du générateur à diverses sondes pourvues d'électrodes permettant de détecter en un site distant des potentiels électriques et/ou d'y délivrer des impulsions de stimulation.

Au moment de l'implantation, les sondes sont mécaniquement reliées et électriquement connectées au générateur par l'intermédiaire de prises enfichables qui sont introduites dans le connecteur du générateur de manière à relier les différents contacts de ces prises à des bornes de connexion homologues du circuit interne du générateur.

Les sondes peuvent être des sondes branchées de façon directe et distincte sur le générateur, aussi bien que de façon indirecte au moyen d'un accessoire intermédiaire reliant les diverses sondes à un connecteur commun, multipolaire, du générateur. En tout état de cause, il s'agit de connecter chacune des sondes à des bornes internes du circuit du générateur qui sont en nombre égal à celui des sondes, ou à celui des électrodes de ces sondes.

L'invention concerne plus particulièrement ceux de ces dispositifs qui permettent à la fois :
- au moyen d'une ou plusieurs "sondes cardiaques", intracardiaques ou épicardiques : de détecter des potentiels de dépolarisation du myocarde ("potentiels cardiaques") et/ou de délivrer au coeur des impulsions électriques de stimulation, défibrillation ou resynchronisation ("impulsions de stimulation cardiaque") - ces fonctions de détection et/ou de délivrance d'impulsions en relation avec le coeur étant ci-après désignées collectivement sous le terme de "thérapie cardiaque". Les documents US 2010/137929 A1, EP 2 402 054 A1, US 6 317 633 B1, FR 2 558 732 A1, US 2012/123496 A1 ou US 2011/224988 A1 décrivent des exemples de tels dispositifs, pouvant comporter une pluralité de sondes cardiaques de détection/stimulation munies d'électrodes implantées dans ou sur le myocarde ; et
- au moyen d'une ou plusieurs "sondes périphériques" implantables sur un "organe périphérique" autre que le coeur : de détecter des potentiels de dépolarisation ("potentiels périphériques") et/ou de délivrer à cet organe des impulsions de stimulation ("impulsions de stimulation périphérique"), différentes de celles délivrées au coeur tant par leur nature que par leur séquencement - ces fonctions de détection et/ou de délivrance d'impulsions de stimulation en relation avec l'organe périphérique étant ci-après désignées collectivement sous le terme de "thérapie périphérique". L'organe périphérique peut être, par exemple et de façon non limitative, un nerf, notamment le nerf vague, le cerveau, un muscle, etc. La thérapie périphérique peut être notamment, et de façon non limitative, une thérapie de stimulation du nerf vague (VNS), de stimulation de la moelle épinière (SCC), de stimulation profonde du cerveau (DBS), de stimulation d'un nerf périphérique (PNS), de stimulation carotidienne (CBS), de stimulation musculaire (MS).
Le EP 2 179 764 A2 décrit un tel dispositif mixte, susceptible de délivrer conjointement deux thérapies de nature différente, à partir de circuits de stimulation respectifs propres, pouvant être éventuellement regroupés au sein d'un même boitier de générateur. Le document US 2013/116743 A1 décrit un dispositif médical implantable comprenant un circuit de thérapie cardiaque et un circuit de thérapie périphérique. Un tel dispositif nécessite au moins deux sondes de nature également différente : une ou plusieurs sonde(s) pour la thérapie cardiaque et une ou plusieurs autre(s) sonde(s) pour la thérapie périphérique. Pour des raisons pratiques, il peut être commode de disposer de connecteurs identiques pour toutes les sondes reliées au générateur, quelle que soit leur fonction. Le fait de disposer de connecteurs identiques, par exemple des connecteurs de type IS-1, permet de simplifier la conception du générateur et des sondes, d'en optimiser la réalisation, d'utiliser des sondes déjà disponibles. Cette manière de procéder introduit dès lors un risque élevé de non-concordance des sondes avec les prises du générateur lors de la procédure d'implantation. De fait, en cas d'erreur de branchement des sondes les impulsions de stimulation cardiaque et de stimulation périphérique délivrées par le générateur seront appliquées à la mauvaise cible (au coeur au lieu de l'organe périphérique et *vice versa*), ou ne seront pas appliquées du tout. Dans tous les cas, ceci implique des risques graves pour le patient, par exemple lorsqu'une thérapie de type périphérique est appliquée au coeur, ce qui peut induire une tachycardie ou même une fibrillation selon l'énergie et la fréquence des impulsions délivrées.

De façon générale, le but de l'invention est de pouvoir bénéficier d'un dispositif médical implantable actif susceptible de délivrer à la fois une thérapie cardiaque et une thérapie périphérique, dispositif qui écarte tout risque de branchement défectueux des sondes ou, à tout le moins, puisse détecter un tel branchement défectueux.

*Selon un premier aspect,* un dispositif est décrit qui, avant de déclencher toute thérapie, vérifie au moment de l'implantation la compatibilité des sondes branchées sur le générateur. En cas d'incompatibilité, le générateur inhibe la délivrance des thérapies périphériques et/ou cardiaques. De préférence, la thérapie inhibée sera en priorité la thérapie périphérique, la thérapie cardiaque pouvant être éventuellement inhibée si la quantité d'énergie délivrée par le générateur de thérapie cardiaque risque d'induire des effets secondaires ou adverses chez le patient si ces impulsions sont appliquées à l'organe périphérique.

*Selon un second aspect,* un dispositif décrit est capable de détecter a *priori* quelle sonde est connectée à quel circuit, par exemple quelle sonde est connectée au circuit de thérapie cardiaque et capable, en réponse, d'assurer automatiquement le couplage correct des sondes aux circuits correspondants, c'est-à-dire de la sonde cardiaque au circuit de thérapie cardiaque et de la sonde périphérique au circuit de thérapie périphérique. On peut ainsi disposer de sondes pourvues de fiches de connexion identiques, sans qu'il soit nécessaire pour le praticien de se soucier de la prise du connecteur sur lequel doit être introduite l'une ou l'autre de ces fiches. En d'autres termes, le dispositif pallie le risque d'une mauvaise compatibilité du branchement des sondes par une aptitude à l'autodétection des sondes et à la configuration automatique du schéma raccordement de ces sondes aux bornes du générateur qui leur sont associées.

On connait divers générateurs capables de détecter automatiquement l'insertion d'une sonde pour pouvoir ensuite activer diverses fonctionnalités, initialiser un certain nombre de paramètres, mémoriser des données de départ à l'implantation. Ces dispositifs procèdent généralement par mesure de l'impédance entre les bornes du connecteur du générateur : en l'absence de sonde cette impédance est très élevée, mais dès l'insertion d'une sonde la valeur décroit au-dessous d'un certain seuil dont le franchissement est détecté pour sortir le stimulateur d'un mode de veille et le placer dans un mode totalement fonctionnel.

La scrutation continue de l'impédance est toutefois pénalisante en termes de consommation et donc de durée de vie de la pile, car elle nécessite à chaque mesure l'injection d'un courant et l'activation de circuits de mesure de la tension correspondante recueillie. Il n'est pour cette raison pas possible d'opérer une scrutation permanente, notamment dans l'emballage commercial de livraison du générateur. De plus, la réalisation d'une mesure d'impédance automatique pour certaines sondes périphériques pourrait être contre-indiquée.

Le EP 1 618 923 A1 (ELA Medical) décrit une autre technique de détection d'une sonde, qui ne nécessite pas la mesure directe et permanente de l'impédance entre les bornes du générateur. Il s'agit seulement de surveiller la consommation du dispositif, toute modification de cette consommation révélant un changement de comportement de la prothèse, typiquement à la suite de la connexion d'une sonde et de l'implantation de celle-ci du fait de : i) la stimulation sur une charge qui n'est plus infinie (comme c'était le cas dans l'emballage d'expédition), ii) la détection de signaux cardiaques activant les filtres numériques dont la consommation dépend du signal en entrée, ainsi que iii) des réveils du microcontrôleur exécutant des instructions logicielles spécifiques sur chaque nouvelle détection. Le dispositif décrit dans ce document permet également de déterminer le type de sonde utilisée - monopolaire ou bipolaire - et d'adapter en conséquence les divers circuits et algorithmes du dispositif de façon automatique. Cette fonction permet d'éviter tout risque d'erreur résultant d'un défaut qui pourrait par exemple entrainer l'application par erreur d'une stimulation bipolaire à une sonde monopolaire.

Toutefois, ce dispositif, qui est fondé sur les spécificités des sondes et signaux cardiaques, n'est pas transposable à un générateur mixte susceptible de délivrer à la fois une thérapie cardiaque et une thérapie périphérique. Les deux cibles (coeur et organe périphérique) sont en effet de nature très différente. Le générateur de thérapie cardiaque pourrait, certes, distinguer une sonde cardiaque bipolaire d'une sonde cardiaque monopolaire, mais ne pourrait pas faire la différence entre une sonde cardiaque et une sonde périphérique, ou entre une sonde périphérique et une absence de sonde. Il pourrait également faire la différence entre une absence de sonde et une présence de sonde (par la mesure d'impédance) sans toutefois être en mesure de savoir si cette sonde est cardiaque ou périphérique.

En outre, en tout état de cause, la technique décrite par le document précité est limitée à la vérification du branchement des sondes et du paramétrage correct du générateur en fonction du type de sonde (monopolaire ou bipolaire), et ne permet pas de rétablir le cas échéant une configuration incorrecte, ni de laisser la faculté au praticien de brancher de manière totalement indifférente des sondes de différentes natures au générateur, en laissant ce dernier établir de façon automatique le schéma de connexion correct, quelle que soit la manière dont les sondes ont été enfichées sur le générateur.

*Selon le premier aspect précité,* le dispositif comprend, de manière en elle-même connue d'après le EP 2 179 764 A2 précité :
- un circuit de thérapie cardiaque, comprenant un module de détection de premiers potentiels et de génération d'impulsions de stimulation, défibrillation et/ou resynchronisation du myocarde ;
- un circuit de thérapie périphérique, comprenant un module de détection de seconds potentiels et de génération d'impulsions de stimulation périphérique ;
- au moins une première borne de connexion, apte et destinée à recevoir une sonde de détection/stimulation implantable dans ou sur le coeur ; et
- au moins une seconde borne de connexion, apte et destinée à recevoir une sonde de détection/stimulation implantable sur ou à proximité d'un organe périphérique situé à distance du coeur.
Chacune desdites première et seconde bornes de connexion est apte à recevoir indifféremment l'une ou l'autre desdites sonde et autre sonde, et le dispositif comporte en outre des moyens de vérification de la configuration de connexion respective desdites sondes auxdites bornes, ces moyens étant aptes à :
a) recueillir un signal sur ladite première borne de connexion et analyser le signal recueilli pour y révéler la présence d'un signal d'origine cardiaque,
b) recueillir un signal sur ladite seconde borne de connexion et analyser le signal recueilli pour y révéler la présence d'un signal d'origine cardiaque,
c) si le critère a) est vérifié et le critère b) n'est pas vérifié, délivrer en réponse une commande d'activation aux circuits de thérapie cardiaque et de thérapie périphérique, et
d) si le critère a) n'est pas vérifié ou le critère b) est vérifié, délivrer en réponse une commande d'inhibition aux circuits de thérapie cardiaque et de thérapie périphérique.

Selon diverses caractéristiques subsidiaires avantageuses :
- les moyens de vérification sont en outre aptes à émettre un signal d'alerte si l'un et/ou l'autre des critères a) et b) n'est pas vérifié ;
- les moyens de vérification sont en outre aptes à émettre un signal de confirmation si l'un et l'autre des critères a) et b) sont vérifiés ;
- les moyens de vérification comprennent des moyens de test de niveau du signal recueilli sur l'une et l'autre des première et seconde borne de connexion, aptes à révéler la présence d'un signal d'origine cardiaque si le niveau du signal recueilli sur cette borne dépasse un seuil donné pendant la durée d'une fenêtre de détection prédéterminée ;
- les moyens de test de niveau du signal comprennent en outre des moyens de test de stabilité des intervalles séparant des événements successifs dudit signal d'origine cardiaque recueilli pendant la durée de la fenêtre de détection, ces moyens de test de stabilité délivrant en sortie un indicateur de stabilité ou d'absence de stabilité. De plus, les moyens de vérification sont en outre aptes à délivrer en réponse une commande d'inhibition aux circuits de thérapie cardiaque et de thérapie périphérique si l'indicateur délivré est un indicateur d'absence de stabilité ;
- les moyens de vérification comprennent des moyens de comparaison des niveaux, déterminés par les moyens de test de niveau, des signaux respectivement recueillis sur les première et seconde bornes. De plus, ces moyens de comparaison sont en outre aptes à délivrer en réponse une commande d'inhibition aux circuits de thérapie cardiaque et de thérapie périphérique si l'écart entre les niveaux respectifs des signaux recueillis sur les première et seconde bornes est inférieur à un écart minimal prédéterminé.

Selon le *second aspect* le dispositif comprend, de manière en elle-même connue d'après le EP 2 179 764 A2 précité :
- un circuit de thérapie cardiaque, comprenant un module de détection de premiers potentiels et de génération d'impulsions de stimulation, défibrillation et/ou resynchronisation du myocarde ;
- un circuit de thérapie périphérique, comprenant un module de détection de seconds potentiels et de génération d'impulsions de stimulation périphérique ;
- au moins une première borne de connexion, apte à recevoir une sonde ; et
- au moins une seconde borne de connexion, apte à recevoir une autre sonde.

L'une des sondes est une sonde de détection/stimulation implantable dans ou sur le coeur et l'autre est une sonde de détection/stimulation implantable sur ou à proximité d'un organe périphérique situé à distance du coeur. Chacune desdites première et seconde bornes de connexion est apte à recevoir indifféremment l'une ou l'autre desdites sonde et autre sonde, et le dispositif comporte en outre des moyens de reconnaissance desdites sondes et de configuration automatique des bornes de connexion, comprenant :
- des moyens discriminateurs, aptes à :
   - recueillir un signal sur l'une des première et seconde bornes de connexion ;
   - analyser le signal recueilli pour y révéler la présence ou l'absence d'un signal d'origine cardiaque ; et
   - délivrer en réponse une identification de celle d'entre les première et seconde bornes de connexion sur laquelle a été révélée la présence d'un signal d'origine cardiaque, et
- des moyens commutateurs, opérant en réponse aux moyens discriminateurs pour :
   - coupler le circuit de thérapie cardiaque à la borne de connexion identifiée par les moyens discriminateurs comme étant celle sur laquelle a été révélée la présence d'un signal d'origine cardiaque, et
   - coupler le circuit de thérapie périphérique à l'autre borne de connexion.

Selon diverses caractéristiques subsidiaires :
- le dispositif comprend en outre des moyens de vérification, aptes à analyser le signal recueilli sur ladite autre borne de connexion, pour y révéler la présence ou l'absence d'un signal d'origine cardiaque et, si un signal d'origine cardiaque est présent sur ladite autre borne de connexion, délivrer en réponse une commande d'inhibition aux moyens commutateurs ;
- les moyens de vérification sont en outre aptes, si un signal d'origine cardiaque est présent sur ladite autre borne de connexion, à émettre en outre en réponse un signal d'alerte d'indétermination ;
- les moyens discriminateurs comprennent des moyens de test de niveau du signal recueilli, aptes à révéler la présence d'un signal d'origine cardiaque si le niveau du signal recueilli sur la borne de connexion dépasse un seuil donné pendant la durée d'une fenêtre de détection prédéterminée ;
- les moyens de test de niveau du signal comprennent en outre des moyens de test de stabilité des intervalles séparant des événements successifs dudit signal cardiaque recueilli pendant la durée de la fenêtre de détection, ces moyens de test de stabilité délivrant en sortie un indicateur de stabilité ou d'absence de stabilité. De plus, les moyens discriminateurs sont en outre aptes à délivrer une commande d'inhibition aux moyens commutateurs si l'indicateur délivré est un indicateur d'absence de stabilité ;
- les moyens discriminateurs comprennent des moyens de comparaison des niveaux, déterminés par les moyens de test de niveau, des signaux respectivement recueillis sur les première et seconde bornes de connexion. De plus, les moyens commutateurs sont aptes à coupler le circuit de thérapie cardiaque à celle des bornes de connexion sur laquelle le niveau de signal le plus élevé a été recueilli, et à coupler le circuit de thérapie périphérique à celle des bornes de connexion sur laquelle le niveau de signal le moins élevé a été recueilli ;
- les moyens de comparaison des niveaux de signal respectifs sont en outre aptes à délivrer une commande d'inhibition aux moyens commutateurs si l'écart entre les niveaux respectifs des signaux recueillis sur les première et seconde bornes de connexion est inférieur à un écart minimal prédéterminé.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une représentation schématique des différents éléments mis en oeuvre par le dispositif, placés dans leur contexte.
La Figure 2 illustre sous forme de schéma par blocs les différentes fonctions mises en oeuvre par le dispositif.
La Figure 3 est un organigramme présentant l'enchaînement des étapes mises en oeuvre par le module de recherche de la sonde cardiaque.
La Figure 4 est un organigramme présentant l'enchaînement des étapes mises en oeuvre par le module de vérification de la compatibilité des sondes.
La Figure 5 est un organigramme présentant l'enchaînement des étapes mises en oeuvre par le module de configuration automatique des sondes assurant le couplage de celles-ci aux bornes des générateurs respectifs concernés.
La Figure 6 est un organigramme homologue de celui de la Figure 4, dans le cas d'une pluralité de sondes sur la voie cardiaque et/ou la voie périphérique.
La Figure 7 est un organigramme présentant l'enchaînement des étapes mises en oeuvre par le module du test du niveau de détection permettant de reconnaitre la présence ou non d'un signal cardiaque dans le signal détecté par la sonde.
La Figure 8 est un organigramme présentant l'enchaînement des étapes mises en oeuvre par le module de test de stabilité du signal cardiaque détecté par le module de test du niveau de détection.

On va maintenant décrire un exemple de réalisation du dispositif de l'invention.

En ce qui concerne ses aspects logiciels, l'invention peut être mise en oeuvre par une programmation appropriée du logiciel de commande d'un dispositif connu comprenant un microprocesseur programmable et des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Le procédé est mis en oeuvre par des moyens principalement logiciels, au moyen d'algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

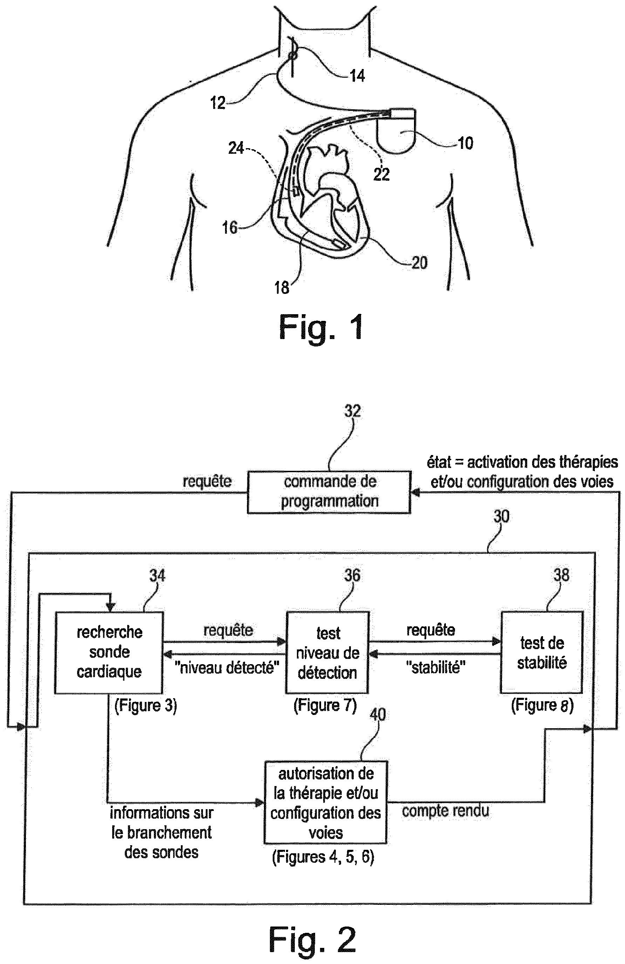

Sur la Figure 1, on a illustré un ensemble comprenant un générateur implantable 10 de thérapie cardiaque et de thérapie périphérique.

Le générateur 10 assure la production d'impulsions de stimulation transmises par une sonde 12 à une électrode appliquée sur un organe périphérique 14, par exemple le nerf vague, cette électrode pouvant être également implantée à proximité de ce nerf ou de l'une de ses branches. Le générateur 10 est également pourvu de circuits de détection/stimulation cardiaque couplés à au moins une électrode d'une sonde 16, par exemple une sonde endocavitaire munie à son extrémité distale 18 d'une électrode en contact avec une paroi du myocarde 20.

Le générateur 10 peut être également muni d'autres sondes que les sondes 12 et 16, par exemple une sonde auriculaire 22 placée dans l'oreillette ou au voisinage de celle-ci et munie à son extrémité distale 24 d'une électrode de détection/stimulation de l'oreillette. La sonde 16, ou les sondes 16 et 22, recueille(nt) des signaux d'électrogramme endocavitaire (EGM) représentatifs de l'activité cardiaque du patient, produits par les dépolarisations du myocarde.

On notera que cette manière d'obtenir un signal d'entrée représentatif de l'activité cardiaque du patient n'est pas limitative, et qu'il est possible d'utiliser en variante ou en complément d'autres signaux pour la mise en oeuvre de l'invention, par exemple des signaux d'accélération endocardiaque (EA) correspondant aux bruits caractéristiques du coeur et permettant de dériver divers paramètres représentatifs de l'activité cardiaque, en particulier comme cela est décrit en détail dans le EP 2 092 885 A1 (ELA Medical).

La Figure 2 illustre sous forme de schéma par blocs les différentes fonctions mises en oeuvre par le dispositif au sein du générateur 10.

Le principe est basé sur la capacité du dispositif à détecter des signaux cardiaques aussi bien sur le canal réservé à la thérapie cardiaque que sur le canal réservé à la thérapie périphérique. La sonde implantée dans le coeur (comme la sonde 16 ou 22 sur la Figure 1) produira un signal cardiaque dont le niveau sera plus élevé que celui produit par une sonde placée hors du coeur (comme la sonde 12 sur la Figure 1). Le dispositif est alors capable d'identifier quelle sonde reçoit le signal le plus élevé, donc quelle est celle qui est implantée dans le coeur.

Selon un premier aspect, avant d'autoriser la délivrance de la thérapie, le dispositif analyse le signal recueilli sur chacune des bornes du générateur (bornes reliées au circuit de thérapie périphérique, et bornes reliées au circuit de thérapie cardiaque) pour déterminer la présence éventuelle d'un signal d'origine cardiaque :
- si la détection d'un signal cardiaque ne peut être établie sur aucune des bornes, il n'est pas possible de savoir quelle sonde est implantée dans le coeur, et un message d'indétermination sera alors produit ;
- s'il est possible d'observer un signal cardiaque sur une seule des deux bornes, ou si le niveau du signal cardiaque détecté est notablement supérieur sur une borne par rapport à l'autre, le système pourra conclure que la sonde couplée à cette borne est une sonde implantée dans le coeur ;
- si un signal d'origine cardiaque est observé sur une sonde qui n'est pas branchée sur la borne du circuit de thérapie cardiaque, alors le dispositif inhibera la délivrance de la thérapie périphérique (et éventuellement de la thérapie cardiaque aussi). Dans le cas contraire, les thérapies périphérique et cardiaque seront autorisées.

Ce principe de vérification de la compatibilité des sondes peut être étendu à un dispositif comportant plusieurs canaux de thérapie cardiaque et/ou plusieurs canaux de thérapie périphérique. La vérification est alors opérée de manière à s'assurer que sur chaque borne reliée au circuit de thérapie cardiaque le signal reçu est au-dessus d'un niveau donné (et donc qu'il s'agit d'un signal cardiaque, présent), tandis que pour chaque borne de thérapie périphérique, le signal reçu est inférieur à un seuil donné. Si l'une au moins des deux conditions précédentes n'est pas vérifiée, alors la délivrance de thérapie cardiaque et périphérique sera inhibée.

Selon un second aspect, les bornes de connexion (cardiaque ou périphérique) ne sont pas attribuées a *priori,* et peuvent recevoir indifféremment les fiches de connexion de l'une ou l'autre des sondes cardiaque ou périphérique.

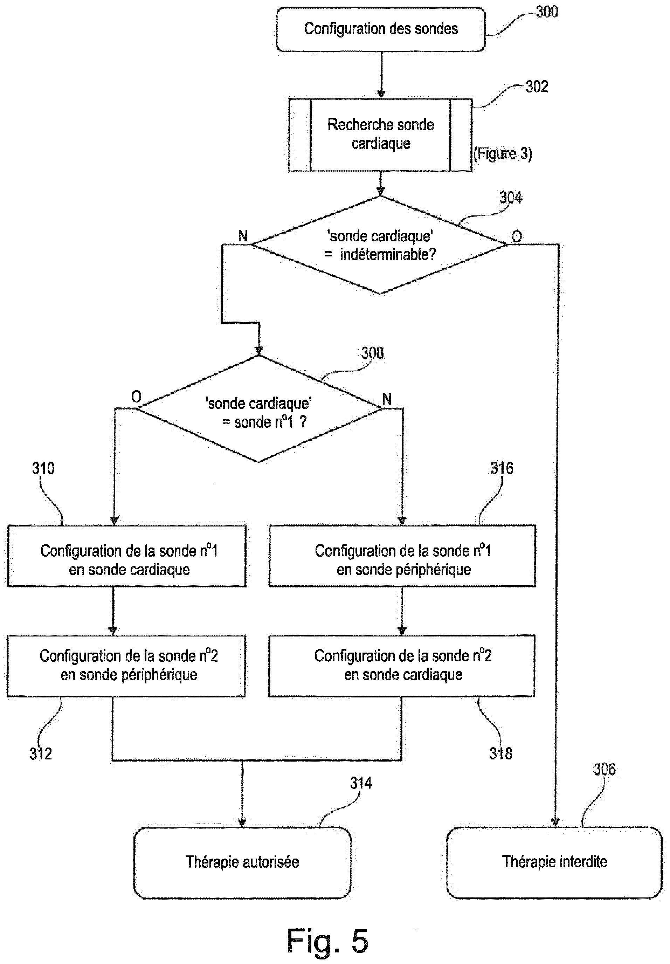

L'attribution des fonctions des bornes est alors établie après auto-identification de la sonde cardiaque : la borne sur laquelle cette sonde est branchée est alors couplée au circuit de thérapie cardiaque, et l'autre sonde est couplée au circuit de thérapie périphérique. Si aucune sonde cardiaque ne peut être trouvée, ou si deux sondes cardiaques sont trouvées, la thérapie n'est pas autorisée et l'algorithme retourne une valeur 'indéterminable' comme paramètre d'identification de la sonde cardiaque, situation qui sera exposée en détail plus bas en relation avec la Figure 5.

Sur la Figure 2 on a représenté sous forme de schéma par blocs les différents modules permettant de réaliser ces fonctionnalités, regroupées dans un module 30 opérant sous le contrôle d'un module 32 de commande de programmation, d'ordre supérieur.

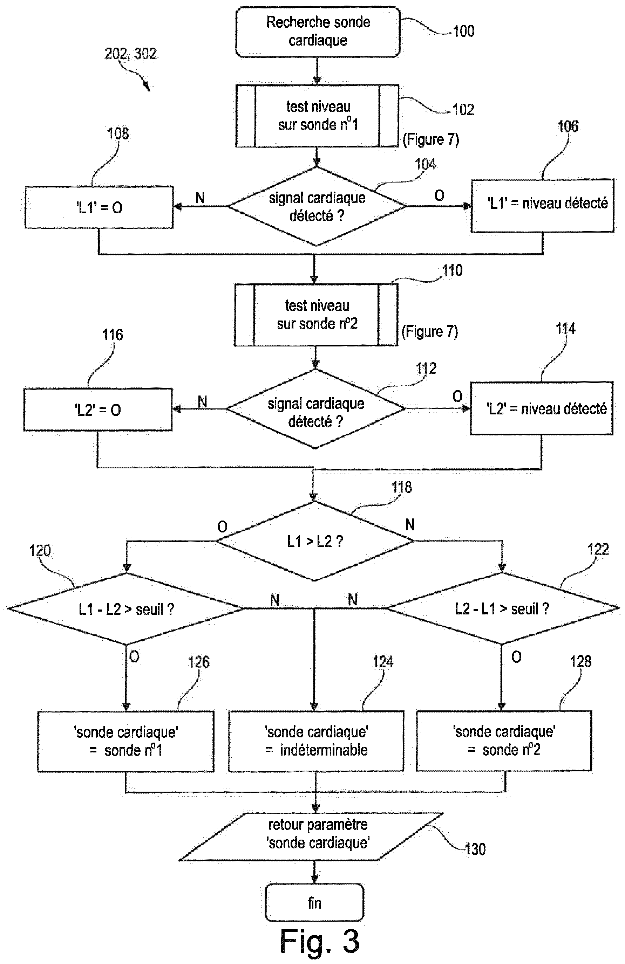

Le module de commande 32 émet une requête en direction du module 30, de manière à rechercher la borne sur laquelle a été branchée une sonde cardiaque (bloc 34, décrit en détail sur la Figure 3).

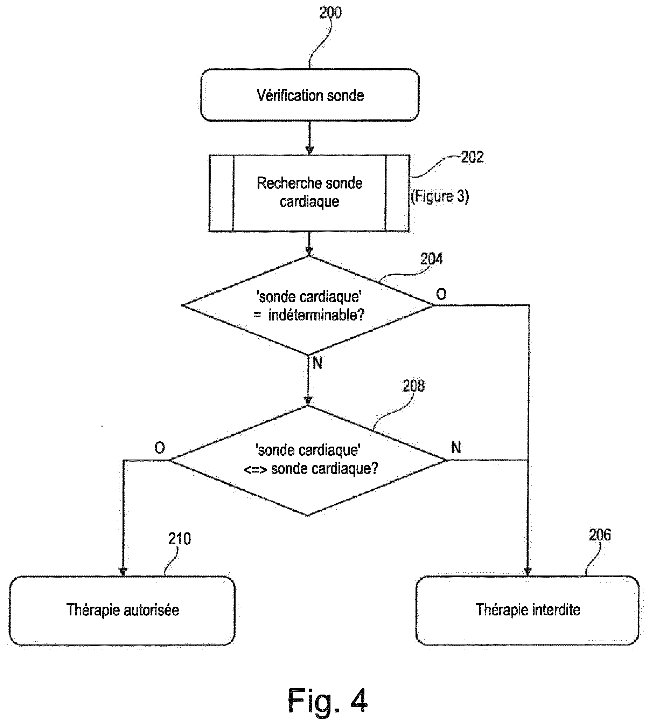

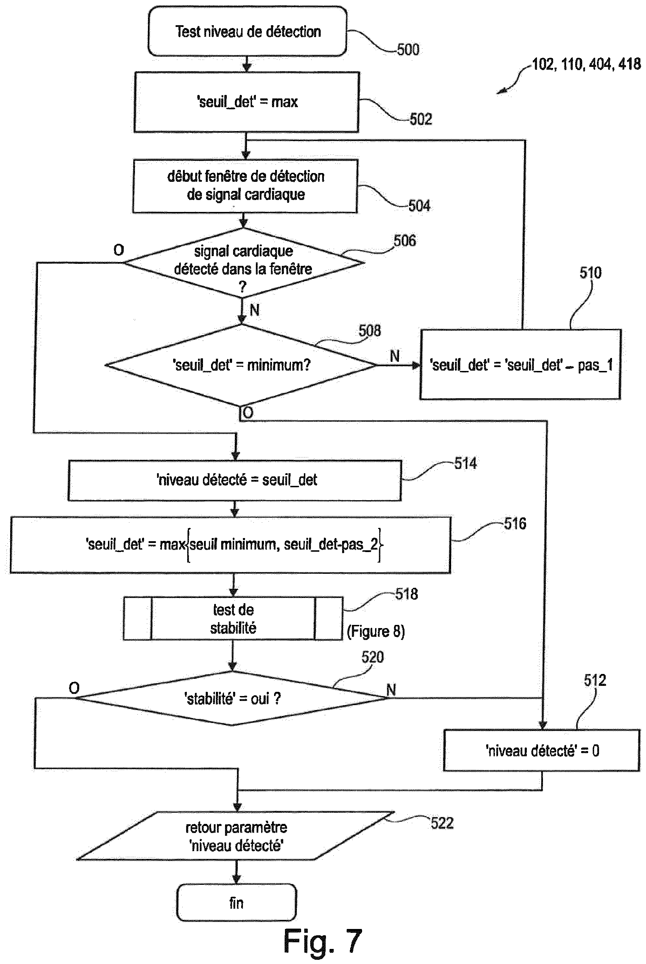

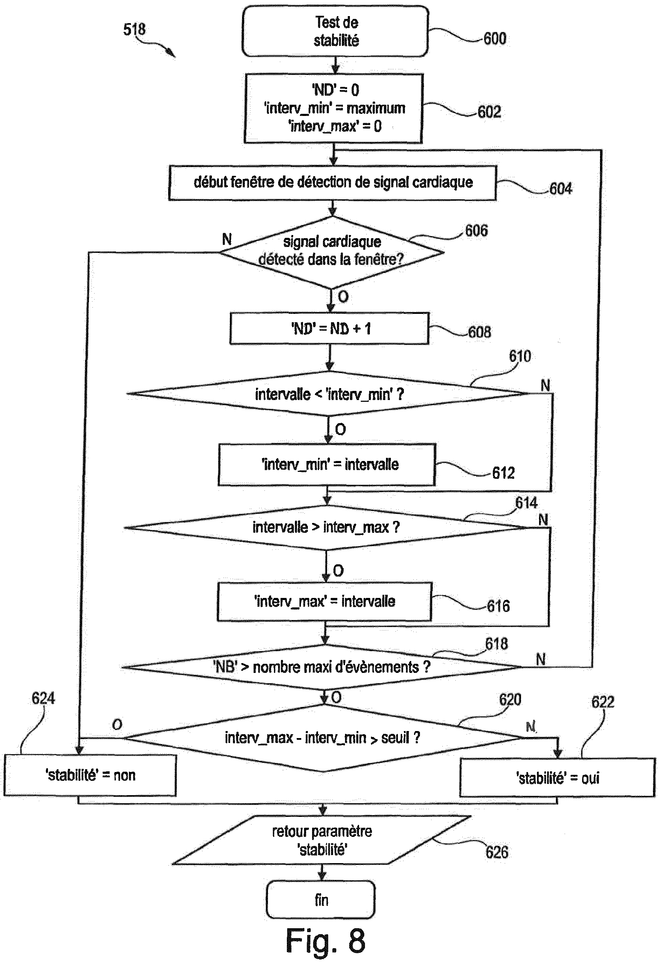

Cette recherche de sonde se fait par échange avec un module de test du niveau de détection (bloc 36, décrit en détail sur la Figure 7) qui retourne un paramètre 'niveau détecté' au module 34. Le module de test du niveau de détection du bloc 36 fait lui-même appel à un module de test de stabilité du signal cardiaque détecté (bloc 38, décrit en détail sur la Figure 8), qui retourne au module de test de niveau un paramètre binaire 'stabilité'. Une fois que la borne sur laquelle est montée la sonde cardiaque a été identifiée (bloc 34), les informations correspondantes sont transmises à un module de vérification de la compatibilité des sondes et d'autorisation de la thérapie, et/ou de configuration automatique des voies (bloc 40, décrit en détail Figures 4, 5 et 6). Le résultat de cette opération (état d'activation ou non des thérapies, et/ou configuration des voies) est alors retourné au module de commande 32 pour poursuite du processus de mise en route du générateur et action appropriée.

La Figure 3 illustre de façon détaillée les étapes mises en oeuvre par le module 34 de recherche de la sonde cardiaque par le dispositif.

L'appel de ce module (bloc 100) déclenche un premier test de niveau de signal sur une première des deux sondes (bloc 102). La manière détaillée dont ce test de niveau est opéré sera décrite plus bas en détail en référence à l'organigramme de la Figure 7.

Si un signal cardiaque a été effectivement détecté sur cette sonde n°1 (bloc 104), alors la valeur du niveau détecté est mémorisée dans une variable 'L1' (bloc 106) ; dans le cas contraire, la variable 'L1' est forcée à zéro (bloc 108).

Les opérations des étapes 102 à 108 sont ensuite répétées de la même façon pour l'autre sonde (blocs 110 à 116), avec pour résultat une seconde variable 'L2' représentative du niveau de signal détecté (ou non) sur la sonde n°2.

L'étape suivante consiste à déterminer celui des niveaux qui est le plus élevé, par comparaison des valeurs des variables L1 et L2 (bloc 118). Toutefois, si l'écart entre les deux valeurs L1 et L2 est, en valeur absolue, inférieur à un seuil donné (blocs 120, 122), on considère qu'il y a indétermination et l'on donne une valeur 'indéterminable' à un paramètre 'sonde cardiaque' (bloc 124). Ce dernier cas peut également correspondre à une situation où deux sondes cardiaques se trouveraient branchées par erreur sur le générateur.

Si l'écart entre les valeurs L1 et L2 est suffisant, le dispositif considère que la sonde cardiaque est la sonde n°1 (bloc 126), dans le cas contraire on considère que la sonde cardiaque est la sonde n°2 (bloc 128). L'algorithme retourne un paramètre 'sonde cardiaque' ayant pour valeur : 'sonde n°1', 'sonde n°2' ou 'indéterminable'.

À partir de l'information 'sonde cardiaque' retournée par le module 34 (correspondant à l'organigramme de la Figure 3), le dispositif opère ensuite (module 40) une vérification de la compatibilité des sondes de manière à autoriser ou non la délivrance d'une thérapie.

Comme illustré Figure 4, cette séquence commence (bloc 200) par un appel au module de recherche de la sonde cardiaque (bloc 202) décrit plus haut en référence à la Figure 3. Si le paramètre 'sonde cardiaque' a la valeur 'indéterminable' (bloc 204) alors on considère que la thérapie ne peut être délivrée : les générateurs d'impulsions sont alors inhibés et un signal d'alerte de thérapie interdite est délivré, par exemple pour transmission à un programmateur externe afin que le praticien puisse prendre une action appropriée en réponse (bloc 206).

Si, en revanche, une sonde cardiaque a pu être effectivement déterminée ('sonde cardiaque' = 'sonde n°1' ou 'sonde n°2'), alors le dispositif vérifie que la sonde correspondante n°1 ou n°2 est bien celle qui est branchée sur la borne du circuit de thérapie cardiaque (bloc 208) : dans l'affirmative, la thérapie est autorisée (bloc 210) ; dans le cas contraire, c'est-à-dire si la sonde cardiaque est branchée sur la borne du circuit de thérapie périphérique, alors le dispositif interdit la délivrance de la thérapie (bloc 206, décrit plus haut).

Dans une autre forme de mise en oeuvre, au lieu de vérifier la compatibilité des sondes, c'est-à-dire de déterminer si les sondes cardiaque et périphérique ont été correctement branchées sur les bornes de connexion respectives, on considère que le branchement des sondes sur les prises du connecteur est un branchement indifférent, le dispositif se chargeant d'identifier les sondes et de coupler ces prises aux générateurs qui leur correspondent.

Cette autre manière de procéder est illustrée sur l'organigramme de la Figure 5.

Le module de configuration automatique des sondes (bloc 300), qui est intégré au module 40 de la Figure 2, opère tout d'abord une recherche de la sonde cardiaque (bloc 302), de la manière décrite en détail plus haut en référence à la Figure 3.

Si le paramètre 'sonde cardiaque' retourné est 'indéterminable' (bloc 304), alors la thérapie est en tout état de cause interdite (bloc 306, comparable au bloc 206 décrit plus haut en référence à la Figure 4).

Si, en revanche, l'une des deux sondes a été identifiée comme étant une sonde cardiaque, par exemple la sonde n°1 (bloc 308), alors la sonde n°1 est configurée en sonde cardiaque, c'est-à-dire couplée au circuit de thérapie cardiaque et l'autre sonde, à savoir la sonde n°2, est configurée en sonde périphérique, c'est-à-dire couplée au circuit de thérapie périphérique (blocs 310 et 312). Dans le cas contraire, c'est la sonde n°1 qui est configurée en sonde périphérique et la sonde n°2, en sonde cardiaque (blocs 316 et 318).

Les sondes ayant été ainsi configurées, la délivrance d'une thérapie est autorisée (bloc 314, comparable au bloc 210 décrit en référence à la Figure 4).

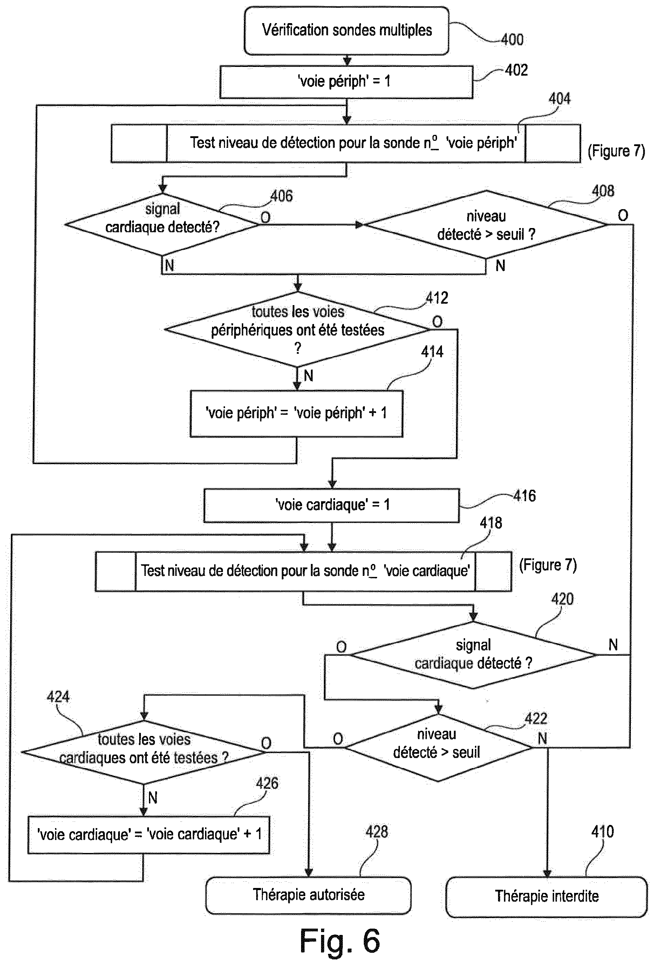

La Figure 6 illustre une généralisation du processus de vérification des sondes, décrit plus haut en référence à la Figure 4 dans le cas de deux sondes, à un nombre quelconque de sondes aussi bien sur la voie cardiaque que sur la voie périphérique.

Ce processus de vérification de la compatibilité d'une multiplicité de sondes (bloc 400) commence par l'initialisation à '1' d'un compteur de voies périphériques (bloc 402), puis un test de niveau de détection est effectué pour la sonde n° 'voie périphérique' (bloc 404). Ce test, semblable à celui des blocs 102 et 110 décrits plus haut, sera décrit en détail ci-après en référence à la Figure 7.

Si, sur cette sonde n° 'voie périphérique', un signal cardiaque est détecté (bloc 406) et que le niveau de ce signal est supérieur à un seuil prédéterminé (bloc 408), alors la thérapie est interdite (bloc 410, comparable aux blocs 206 et 306 décrits plus haut) : en effet, sur une borne censée recevoir une sonde périphérique, un niveau de signal cardiaque élevé indique que la sonde en question a été implantée par erreur dans le coeur.

Dans le cas contraire, c'est-à-dire si aucun signal cardiaque n'a été détecté sur la sonde n° 'voie périphérique', ou si le niveau du signal détecté est très faible, alors le processus est répété pour les sondes connectées aux autres voies périphériques, de manière itérative (blocs 412 et 414).

Une fois que toutes les voies périphériques ont été explorées, le processus est répété de la même façon pour les diverses voies cardiaques, par les étapes 416, 418, 420, 422, 424 et 426 homologues des étapes 402, 404, 406, 408, 412 et 414 décrites plus haut.

Après avoir ainsi exploré toutes les voies périphériques et toutes les voies cardiaques, si la compatibilité est avérée, alors la thérapie est autorisée (bloc 428, comparable aux blocs 210 et 314 décrits plus haut).

L'organigramme de la Figure 7 illustre les diverses étapes du test de niveau de détection des étapes 102, 110, 404 et 418 des organigrammes précédents.

Au début de ce test (bloc 500), un paramètre 'seuil_det' est initialisé à une valeur maximale prédéfinie (bloc 502). Une fenêtre de détection du signal cardiaque est alors ouverte (bloc 504) de manière à déterminer la présence ou l'absence d'un signal cardiaque dans cette fenêtre (bloc 506). Dans la négative, le paramètre 'seuil_det' est réduit d'un pas ('pas_1'), tant qu'il n'a pas atteint une valeur minimum prédéfinie (blocs 508 et 510). Si, à l'étape 508, le seuil de détection a atteint la valeur minimale prédéterminée, alors la valeur 'niveau détecté' est forcée à zéro (bloc 512) et il est mis fin au processus : ceci signifie que même avec un seuil très faible, il n'est pas possible de détecter un signal cardiaque, et que l'on se trouve donc vraisemblablement en présence d'un signal provenant d'une sonde implantée sur ou à proximité d'un organe périphérique, et non dans le coeur.

Si à l'étape 506 un signal cardiaque est observé, alors la valeur de 'seuil_det' est mémorisée dans un paramètre 'niveau détecté' (bloc 514), puis 'seuil_det' est forcé à une valeur égale à la plus élevée soit du minimum, soit de la valeur courante 'seuil_det' réduite d'un pas ('pas_2') (bloc 516).

Le processus procède alors à un test de stabilité du rythme cardiaque, décrit ci-après en détail en référence à la Figure 8 (bloc 518). Si la stabilité est avérée (bloc 520), alors le test retourne le paramètre 'niveau détecté' déterminé conformément à l'algorithme (bloc 522).

Si, à l'étape 520, la stabilité du rythme cardiaque n'est pas avérée, alors le paramètre 'niveau détecté' est forcé à zéro (bloc 512) : en effet, bien que l'on détecte un signal de niveau supérieur au seuil de détection, ce signal n'est pas stable, ce qui laisse suspecter une anomalie qui devra être prise en compte.

La Figure 8 illustre les diverses étapes du test de stabilité mentionné plus haut à l'étape 518 de la Figure 7.

L'appel de cette procédure (bloc 600) commence par initialiser un certain nombre de paramètres, à savoir un compteur d'événements 'NB' initialisé à zéro, un paramètre d'intervalle minimal 'interv_min' initialisé à une valeur maximale prédéfinie, et un paramètre d'intervalle maximal 'interv_max' initialisé à zéro.

Une fenêtre de détection de signal cardiaque est ouverte (bloc 604) pour détecter la présence du signal cardiaque dans cette fenêtre (bloc 606). Lorsque le signal est détecté, par exemple une onde P, le compteur d'événements 'NB' est incrémenté d'une unité (bloc 608) et l'intervalle mesuré est comparé aux limites 'interv_min' et "interv_max' et forcé à l'une ou l'autre de ces valeurs si la limite est atteinte (blocs 610, 612, 614 et 616).

Le processus est répété de manière itérative jusqu'à atteindre un nombre maximal prédéterminé d'événements (bloc 618). Les étapes 610 à 616 itérées opèrent une recherche du minimum et du maximum de l'intervalle de tous les événements successivement détectés, la valeur de minimum/maximum étant mise à jour éventuellement à chaque événement, par rapport à l'évènement précédent.

Si l'écart entre l'intervalle maximal 'interv_max' et l'intervalle minimal 'interv_min' est inférieur à un seuil prédéterminé (bloc 620) on considère que le rythme est stable et l'on donne la valeur 'oui' au paramètre 'stabilité' (bloc 622). Dans le cas contraire, on donne la valeur 'non' au paramètre 'stabilité' (bloc 624). Ce paramètre 'stabilité' est alors retourné au module de test du niveau de détection (module 36 de la Figure 2, dont le processus est explicité à la Figure 7), pour poursuite des opérations.

## Revendications

1. Un dispositif médical implantable actif (10), comprenant :
- un circuit de thérapie cardiaque, comprenant un module de détection de premiers potentiels et de génération d'impulsions de stimulation, défibrillation et/ou resynchronisation du myocarde ;
- un circuit de thérapie périphérique, comprenant un module de détection de seconds potentiels et de génération d'impulsions de stimulation périphérique ;
- au moins une première borne de connexion, apte et destinée à recevoir une sonde (16, 22) de détection/stimulation implantable dans ou sur le coeur (20) ; et
- au moins une seconde borne de connexion, apte et destinée à recevoir une sonde (12) de détection/stimulation implantable sur ou à proximité d'un organe périphérique (14) situé à distance du coeur,
où chacune desdites première et seconde bornes de connexion est apte à recevoir indifféremment l'une ou l'autre desdites sondes (16, 22 ; 12), et en ce qu'il comporte des moyens (30) de vérification de la configuration de connexion respective desdites sondes auxdites bornes, ces moyens étant aptes à :
a) recueillir un signal sur ladite première borne de connexion et analyser (102-106 ; 404 ; 416) le signal recueilli pour y révéler la présence d'un signal d'origine cardiaque,
b) recueillir un signal sur ladite seconde borne de connexion et analyser (110-114 ; 418-426) le signal recueilli pour y révéler la présence d'un signal d'origine cardiaque, **caractérisé en ce que** les moyens sont aptes à
c) si le critère a) est vérifié et le critère b) n'est pas vérifié, délivrer en réponse une commande d'activation (210 ; 428) aux circuits de thérapie cardiaque et de thérapie périphérique, et
d) si le critère a) n'est pas vérifié ou le critère b) est vérifié, délivrer en réponse une commande d'inhibition (206 ; 410) aux circuits de thérapie cardiaque et de thérapie périphérique.

2. Le dispositif de la revendication 1, dans lequel les moyens de vérification comprennent des moyens d'émission d'un signal d'alerte si l'un et/ou l'autre des critères a) et b) n'est pas vérifié.

3. Le dispositif de la revendication 1, dans lequel les moyens de vérification comprennent des moyens d'émission d'un signal de confirmation si l'un et l'autre des critères a) et b) sont vérifiés.

4. Le dispositif de la revendication 1, dans lequel les moyens de vérification comprennent des moyens de test de niveau (520-522) du signal recueilli sur l'une et l'autre des la première et seconde borne de connexion, aptes à révéler la présence d'un signal d'origine cardiaque si le niveau du signal recueilli sur cette borne dépasse un seuil donné ('seuil_det') pendant la durée d'une fenêtre de détection prédéterminée.

5. Le dispositif de la revendication 4, dans lequel :
- les moyens de test de niveau du signal comprennent en outre des moyens de test de stabilité (602-626) des intervalles séparant des évènements successifs dudit signal d'origine cardiaque recueilli pendant la durée de la fenêtre de détection, ces moyens de test de stabilité délivrant en sortie un indicateur ('stabilité') de stabilité ou d'absence de stabilité, et
- les moyens de vérification sont en outre aptes à délivrer en réponse une commande d'inhibition aux circuits de thérapie cardiaque et de thérapie périphérique si l'indicateur délivré est un indicateur d'absence de stabilité.

6. Le dispositif de la revendication 4, dans lequel :
- les moyens de vérification comprennent des moyens de comparaison (118-128) des niveaux, déterminés par les moyens de test de niveau, des signaux respectivement recueillis sur les première et seconde bornes, et
- ces moyens de comparaison sont en outre aptes à délivrer en réponse une commande d'inhibition aux circuits de thérapie cardiaque et de thérapie périphérique si l'écart entre les niveaux respectifs des signaux recueillis sur les première et seconde bornes est inférieur à un écart minimal ('seuil') prédéterminé.

## Patentansprüche

1. Aktive implantierbare medizinische Vorrichtung (10), umfassend:
- eine Herztherapieschaltung, umfassend ein Modul zur Erfassung von ersten Potentialen und zur Erzeugung von Impulsen zur Stimulation, Defibrillation, und/oder Resynchronisierung des Herzmuskels;
- eine periphere Therapieschaltung, umfassend ein Modul zur Erfassung von zweiten Potentialen und zur Erzeugung von Impulsen zur peripheren Stimulation;
- mindestens eine erste Anschlussklemme, die dazu geeignet und bestimmt ist, eine im oder am Herzen (20) implantierbare Erfassungs-/Stimulationssonde (16, 22) aufzunehmen; und
- mindestens eine zweite Anschlussklemme, die dazu geeignet und bestimmt ist, eine im oder in der Nähe eines vom Herzen entfernten peripheren Organs (14) implantierbare Erfassungs-/Stimulationssonde (12) aufzunehmen,
wobei jede der ersten und zweiten Anschlussklemmen dazu geeignet ist, gleichermaßen die eine oder andere der Sonden (16, 22; 12) aufzunehmen,
und umfassend Mittel (30) zur Überprüfung der Konfiguration der jeweiligen Verbindung der Sonden mit den Klemmen, wobei diese Mittel dazu geeignet sind:
a) an der ersten Anschlussklemme ein Signal aufzufangen und das aufgefangene Signal zu analysieren (102-106; 404; 416), um das Vorhandensein eines Herzsignals zu bestimmen,
b) an der zweiten Anschlussklemme ein Signal aufzufangen und das aufgefangene Signal zu analysieren (110-114; 418-426), um das Vorhandensein eines Herzsignals zu bestimmen,
**dadurch gekennzeichnet, dass** die Mittel dazu geeignet sind:
c) wenn das Kriterium a) erfüllt ist und das Kriterium b) nicht erfüllt ist, in Antwort, an die Herztherapieschaltung und die periphere Therapieschaltung einen Aktivierungsbefehl (210; 428) auszugeben, und
d) wenn das Kriterium a) nicht erfüllt ist oder das Kriterium b) erfüllt ist, in Antwort, an die Herztherapieschaltung und die periphere Therapieschaltung einen Unterdrückungsbefehl (206; 410) auszugeben.

2. Vorrichtung nach Anspruch 1, wobei die Überprüfungsmittel Mittel umfassen, die ein Warnsignal ausgeben, wenn die Kriterien a) und b) nicht erfüllt sind.

3. Vorrichtung nach Anspruch 1, wobei die Überprüfungsmittel Mittel umfassen, die ein Bestätigungssignal ausgeben, wenn beide Kriterien a) und b) erfüllt sind.

4. Vorrichtung nach Anspruch 1, wobei die Überprüfungsmittel Mittel zur Prüfung des Pegels (520-522) des an der ersten und zweiten Anschlussklemme aufgefangenen Signals umfassen, die dazu geeignet sind, das Vorhandensein eines Herzsignals zu bestimmen, wenn der Pegel des an dieser Anschlussklemme aufgefangenen Signals während eines vorbestimmten Erfassungszeitfensters eine bestimmte Schwelle ("seuil-det") überschreitet.

5. Vorrichtung nach Anspruch 4, wobei:
- die Mittel zur Prüfung des Signalpegels außerdem Mittel zur Prüfung der Stabilität (602-626) der Zeitintervalle umfassen, welche aufeinanderfolgende Ereignisse des Herzsignals trennen, welcher während des Erfassungszeitfensters aufgefangen wird, wobei diese Stabilitäts-Prüfmittel einen Stabilitäts- oder Nicht-Stabilitäts-Indikator ("stabilite") ausgeben, und
- die Überprüfungsmittel außerdem dazu geeignet sind, wenn der ausgegebene Indikator ein Nicht-Stabilitäts-Indikator ist, in Antwort, an die Herztherapieschaltung und die periphere Therapieschaltung einen Unterdrückungsbefehl auszugeben.

6. Vorrichtung nach Anspruch 4, wobei:
- die Überprüfungsmittel Mittel zum Vergleich (118-128) der durch die Mittel zur Prüfung des Pegels bestimmten Pegel der jeweils an der ersten und zweiten Klemme erfassten Signale umfassen, und
- diese Vergleichsmittel außerdem dazu geeignet sind, wenn der Unterschied zwischen den jeweiligen Pegeln der an der ersten und zweiten Klemme aufgefangenen Signale kleiner als ein vorbestimmter minimaler Unterschied ("seuil") ist, in Antwort, an die Herztherapieschaltung und die periphere Therapieschaltung einen Unterdrückungsbefehl auszugeben.

## Claims

1. An active implantable medical device (10) comprising:
- a cardiac therapy circuit comprising a module for detecting first potentials and generating myocardial stimulation, defibrillation and/or resynchronization pulses;
- a peripheral therapy circuit comprising a module for detecting second potentials and generating peripheral stimulation pulses;
- at least one first connection terminal, adapted and designed to receive a detection/stimulation lead (16, 22) that is implantable in or on the heart (20); and
- at least one second connection terminal, adapted and designed to receive a detection/stimulation lead (12) that is implantable on or close to a peripheral organ (14) located at a distance from the heart,
wherein each of said first and second connection terminals is adapted to equally receive one or the other of said leads (16, 22; 12),
and comprising means (30) for verifying the respective connection configuration of said leads to said terminals, the means being adapted to:
a) collect a signal from said first connection terminal and analyze (102-106; 404; 416) the collected signal to reveal the presence of a cardiac signal,
b) collect a signal from said second connection terminal and analyze (110-114; 418-426) the collected signal to reveal the presence of a cardiac signal,
**characterized in that** the means are adapted:
c) if criterion a) is verified and criterion b) is not verified, to issue, as a response, an activation order (210; 428) to the cardiac therapy circuit and the peripheral therapy circuit, and
d) if criterion a) is not verified or criterion b) is verified, to issue, as a response, an inhibition order (206; 410) to the cardiac therapy circuit and the peripheral therapy circuit.

2. The device according to claim 1, wherein the verification means comprise means for emitting a warning signal if one and/or the other of criteria a) and b) is not verified.

3. The device according to claim 1, wherein the verification means comprise means for emitting a confirmation signal if one and/or the other of criteria a) and b) are verified.

4. The device according to claim 1, wherein the verification means comprise means for testing the level (520-522) of the signal collected from each of the first and second connection terminals, said testing means being adapted to reveal the presence of a cardiac signal if the level of the signal collected from that terminal exceeds a given threshold ("seuil_det") during a predetermined detection time window.

5. The device according to claim 4, wherein:
- the means for testing the level of the signal further comprise means for testing the stability (602-626) of the intervals separating successive events of said cardiac signal collected during the detection time window, these means for testing the stability issuing a stability or lack of stability indicator ("stabilité"), and
- if the issued indicator is a lack of stability indicator, the verification means are further adapted to issue, as a response, an inhibition order to the cardiac therapy circuit and peripheral therapy circuit

6. The device according to claim 4, wherein:
- the verification means comprise means (118-128) for comparing the levels, determined by the level testing means, respectively collected from the first and second terminals, and
- if the difference between the respective levels of the signals collected from the first and second terminals is smaller than a predetermined minimal difference ("seuil"), these verification means are further adapted to issue, in response, an inhibition order to the cardiac therapy circuit and the peripheral therapy circuit.
